# FASCICULE DE BREVET EUROPEEN

(11) **EP 2 250 187 B1**
(45) Date de publication et mention de la délivrance du brevet: **12.12.2012**
(21) Numéro de dépôt: 09720065.3
(22) Date de dépôt: 04.02.2009
(51) Int. Cl.: C07K 7/06, A61K 38/08, A61K 8/64

(54) **PEPTIDE ET COMPOSITION COSMETIQUE ET/OU PHARMACEUTIQUE LE CONTENANT**
PEPTID UND DIESES ENTHALTENDE KOSMETISCHE UND/ODER PHARMAZEUTISCHE ZUSAMMENSETZUNG
PEPTIDE AND COSMETIC AND/OR PHARMACEUTICAL COMPOSITION CONTAINING THE SAME

(30) Priorité: 06.02.2008 FR 0800611
(43) Date de publication de la demande: 17.11.2010
(73) Titulaire: Société d'Extraction des Principes Actifs SA (Vincience), 06410 Biot (FR)
(72) Inventeur: DAL FARRA, Claude, Kerhonkson New York 12446 (US); DOMLOGE, Nouha, F-06560 Valbonne (FR); BOTTO, Jean-Marie, F-06560 Valbonne (FR)
(74) Mandataire: Macquet, Christophe
(86) Numéro de dépôt international: PCT/FR2009/000123
(87) Numéro de publication internationale: WO 2009/112708

(56) Documents cités:
- WO-A-03/077936
- WO-A-2007/057449
- DAL FARRA CLAUDE ET AL: "Anti-aging effects observed in new synthetic peptide" JOURNAL OF THE AMERICAN ACADEMY OF DERMATOLOGY, vol. 56, no. 2, février 2007 (2007-02), page AB25, XP002497802

### SEQUENCE LISTING

<110> société d'Extraction des Principes Actifs (vincience)
<120> NOUVEAU PEPTIDE ET COMPOSITION COSMETIQUE ET/OU PHARMACEUTIQUE LE CONTENANT
<130> Bv 08-105
<150> FR0800611
   <151> 2008-02-06
<160> 10
<170> PatentIn version 3.3
<210> 1
   <211> 4
   <212> PRT
   <213> Synthetic Peptides
<400> 1
   Arg Phe Leu Arg
   1
<210> 2
   <211> 5
   <212> PRT
   <213> Synthetic Peptides
<400> 2
   Leu Arg Phe Leu Arg
   1               5
<210> 3
   <211> 6
   <212> PRT
   <213> Synthetic Peptides
<400> 3
   Arg Leu Arg Phe Leu Arg
   1 5
<210> 4
   <211> 7
   <212> PRT
   <213> Synthetic Peptides
<400> 4
   Ile Arg Leu Arg Phe Leu Arg
   1 5
<210> 5
   <211> 7
   <212> , PRT
   <213> Synthetic Peptides
<220>
   <221> MOD_RES
   <222> (7)..(7)
   <223> AMIDATION
<400> 5
   Ile Arg Leu Arg Phe Leu Arg
   1 5
<210> 6
   <211> 7
   <212> PRT
   <213> Synthetic Peptides
<400> 6
   Ile Lys Leu Arg Phe Leu His
   1 5
<210> 7
   <211> 7
   <212> PRT
   <213> Synthetic Peptides
<220>
   <221> MOD_RES
   <222> (7)..(7)
   <223> AMIDATION
<400> 7
   Ile Lys Leu Arg Phe Leu His
   1 5
<210> 8
   <211> 7
   <212> PRT
   <213> Synthetic Peptides
<400> 8
   Ile His Leu Lys Phe Leu Arg
   1 5
<210> 9
   <211> 7
   <212> PRT
   <213> Synthetic Peptides
<220>
   <221> MOD_RES
   <222> (7)..(7)
   <223> AMIDATION
<400> 9
   Ile His Leu Lys Phe Leu Arg
   1 5
<210> 10
   <211> 7
   <212> PRT
   <213> synthetic Peptides
<400> 10
   Ile Lys Leu His Phe Leu Lys
   1 5

## Description

La présente invention se situe dans le domaine cosmétique et pharmaceutique, et plus particulièrement dans le domaine de la dermatologie. La présente invention concerne un peptide de formule générale (I) : R₁-(AA)ₙ-Ile-X₁-Leu-X₂-Phe-Leu-X₃-(AA)ₚ-R₂, destiné à stimuler la synthèse des principales protéines de la matrice extracellulaire de la peau. La présente invention concerne également une composition cosmétique, nutraceutique ou pharmaceutique, comprenant un peptide de formule générale (I) en tant qu'agent actif. L'invention est également relative à l'utilisation de ce nouvel agent actif dans une composition cosmétique ou nutraceutique destinée à prévenir et/ou lutter contre les manifestations du vieillissement cutané, à stimuler la régénération cutanée et à restructurer du derme. L'invention est également relative à l'utilisation de ce nouvel agent actif, par voie orale ou topique, pour la réalisation d'une composition pharmaceutique, et notamment dermatologique, destinée à améliorer la cicatrisation. L'invention est également relative à l'utilisation de ce nouvel agent actif, par voie orale ou topique, pour la réalisation d'une composition pharmaceutique buccale destinée à protéger ou à traiter les muqueuses buccales et les gencives (tissus conjonctifs non minéralisés du parodonte).

La peau est un organe vital composé de plusieurs couches (derme, couches prolifératives et *stratum corneum)* qui recouvre toute la surface du corps et assure essentiellement une fonction de barrière vis-à-vis du milieu extérieur. Constitué d'eau, de fibres d'élastine et de fibres de collagène enveloppées dans une matrice interstitielle de proteoglycanes, le derme est le tissu de soutien de la peau. Le derme est vascularisé, ce qui lui permet d'apporter à l'épiderme énergie et nutriments et de jouer un rôle primordial dans la thermorégulation et la cicatrisation. Les fibroblastes sont les principales cellules du derme. Ils sont essentiellement localisés dans le derme papillaire proche de l'épiderme, et peu représentés dans le derme profond dit derme réticulaire. Ils sont spécialisés dans la synthèse de deux types de fibres protéiques : les fibres de collagène et les fibres d'élastine, constituants de la matrice extracellulaire. Les premières constituent 70 % des fibres dermiques et lui confèrent sa résistance aux tensions et aux tractions, tandis que les secondes lui donnent ses propriétés élastiques.

Le vieillissement cutané est un phénomène complexe qui est dû à de nombreux facteurs endogènes et exogènes. Cliniquement, on observe l'apparition de rides et ridules, une perte de l'élasticité cutanée, un relâchement des tissus cutanés et sous-cutanés. D'un point de vue histologique, le vieillissement cutané se caractérise, entre autre, par une diminution globale de l'épaisseur de la peau, et en particulier du derme, une diminution du renouvellement cellulaire, une diminution de la densité et de l'organisation de fibres de collagène et d'élastine de la matrice extracellulaire.
Ainsi, par manifestations cutanées du vieillissement, on entend toutes modifications de l'aspect extérieur de la peau et des phanères dues au vieillissement comme, par exemple, les rides et ridules, mais également une diminution des propriétés élastiques et de la fermeté de la peau, un ralentissement de la cicatrisation. Ces modifications peuvent être dues au vieillissement intrinsèque ou à une exposition aux rayonnements ultraviolets (UV).

De nombreuses voies de recherche sont proposées pour lutter contre le vieillissement, parmi lesquelles la protection contre les stress environnementaux (soleil, pollutions...), l'activation de la régénération cellulaire, le renforcement de la matrice extracellulaire (collagène et élastine). Récemment, des études ont montré l'importance de l'adhésion entre les cellules épidermiques (kératinocytes), cette adhésion étant requise pour la cohésion de l'épiderme mais aussi pour la communication cellulaire dans le traitement des peaux âgées.

Les professionnels de la santé et de la cosmétique sont donc en permanence à la recherche de principes actifs réellement efficaces et ayant un large spectre d'action. De plus, ces actifs se doivent d'avoir une forte compatibilité dermatologique de façon à ce que chez tous les utilisateurs, même les plus sensibles, il n'y ait aucune réaction d'irritation. Il subsiste donc le besoin de développer des actifs dermatologiques et/ou cosmétiques ayant une très bonne efficacité ainsi qu'un large spectre d'action au niveau de la peau, des phanères et des muqueuses.

La recherche d'agents actifs capables de prévenir ou de lutter contre les manifestations du vieillissement cutané, ou encore de limiter les dommages causés par les agressions extérieures, telles que les expositions prolongées aux UV, est une préoccupation importante de la recherche médicale et de la cosmétique. A cet égard, il a été proposé des principes actifs de nature peptidique, capables d'agir sur la matrice extracellulaire (FR 2837098, FR 2827174, FR 2827170, US 5696229, EP 1112057). Mais à ce jour, aucun peptide tel que divulgué dans la présente invention n'a encore été décrit.

Les inventeurs ont en effet mis en évidence une activité cosmétique et thérapeutique, notamment dermatologique, d'un peptide. Il a en particulier été mis en évidence que ce peptide, lorsqu'il est appliqué sur la peau, stimule la synthèse de molécules de la matrice extracellulaire telles que la fibronectine, le collagène de type 1 et le collagène de type III, les kératines. Ces propriétés peuvent avantageusement être mises à profit pour préparer des compositions capables de prévenir et de lutter contre les manifestations du vieillissement cutané, de stimuler la régénération cutanée et de restructurer le derme.

On entend par « application topique », le fait d'appliquer ou d'étaler l'agent actif selon l'invention, ou une composition le contenant, à la surface de la peau ou d'une muqueuse.

On entend par « cosmétiquement ou dermatologiquement acceptable », que l'agent actif selon l'invention, ou une composition le contenant, est approprié pour entrer en contact avec la peau ou une muqueuse sans provoquer de réactions de toxicité ou d'intolérance.

On entend par « nutraceutique », une méthode de soin de la peau, qui implique l'administration d'agents actifs par des voies autres que la voie topique, à des sujets en bonne santé qui veulent améliorer ou entretenir leur apparence physique.

On entend par « restructuration du derme », une amélioration de l'organisation, de la densité et de l'orientation des fibres de collagène.

Par l'expression « biologiquement actif », on entend « qui possède une activité *in vivo* ou *in vitro* caractéristique de l'activité de l'agent actif selon l'invention ».

Ainsi, l'invention a pour objet premier un peptide dont le nombre d'acides aminés est compris entre 3 et 7, et qui possède une séquence qui répond à la formule générale (I) :

R₁-(AA)ₙ-Ile-X₁-Leu-X₂-Phe-Leu-X₃-(AA)ₚ-R₂

Dans laquelle,
X₁, X₂ ou X₃ est l'arginine, la lysine ou l'histidine,
AA représente un acide aminé quelconque, autre que l'asparagine ou la glutamine, ou un de ses dérivés, et n et p sont des nombres entiers compris entre 0 et 4,
R₁ représente la fonction amine primaire de l'acide aminé N-terminal, libre ou substituée par un groupement protecteur pouvant être choisi parmi un groupement acétyle, un groupement benzoyle, un groupement tosyle ou un groupement benzyloxycarbonyle,
R₂ représente le groupe hydroxyle de la fonction carboxyle de l'acide aminé C-terminal, libre ou substitué par un groupement protecteur pouvant être choisi parmi une chaîne alkyle de C₁ à C₂₀, ou un groupement NH2, NHY ou NYY avec Y représentant une chaîne alkyle de C₁ à C₄,
Ladite séquence de formule générale (I) peut comprendre des substitutions des acides aminés X₁ à X₃ par d'autres acides aminés chimiquement équivalents.

Selon une méthode de réalisation de l'invention tout particulièrement préférée, le peptide biologiquement actif est de séquence :
(SEQ ID n°1) Arg-Phe-Leu-Arg
(SEQ ID n°2) Leu-Arg-Phe-Leu-Arg
(SEQ ID n°3) Arg-Leu-Arg-Phe-Leu-Arg
(SEQ ID n°4) Ile-Arg-Leu-Arg-Phe-Leu-Arg
(SEQ ID n°5) Ile-Arg-Leu-Arg-Phe-Leu-Arg -NH₂
(SEQ ID n°6) Ile-Lys-Leu-Arg-Phe-Leu-His
(SEQ ID n°7) Ile-Lys-Leu-Arg-Phe-Leu-His-NH₂
(SEQ ID n°8) Ile-His-Leu-Lys-Phe-Leu-Arg
(SEQ ID n°9) Ile-His-Leu-Lys-Phe-Leu-Arg -NH₂
(SEQ ID n°10) Ile-Lys-Leu-His-Phe-Leu-Lys

Selon un mode de réalisation particulièrement intéressant, le peptide biologiquement actif correspond à la séquence SEQ ID n°4.

Selon un autre mode de réalisation particulièrement intéressant, le peptide biologiquement actif correspond à la séquence SEQ ID n°5.

L'invention concerne aussi des formes homologues de ces séquences. Le terme « homologue » désigne, selon l'invention, toute séquence peptidique identique à au moins 50%, ou de préférence au moins 80%, et encore plus préférentiellement à au moins 90% de ladite séquence peptidique, choisie parmi les séquences SEQ ID n° 1 à SEQ ID n° 10. Par « séquence peptidique identique à au moins X% », on entend désigner un pourcentage d'identité entre les résidus d'acides aminés des deux séquences à comparer, obtenu après l'alignement optimal des deux séquences. L'alignement optimal est obtenu à l'aide d'algorithmes d'homologies locales tels que ceux utilisés par les logiciels informatiques BLAST P ou T BLAST N disponibles sur le site NCBI.

Le terme « homologue » peut également désigner un peptide qui diffère de la séquence d'un peptide de séquence SEQ ID n° 1 à SEQ ID n° 10 par la substitution d'acides aminés chimiquement équivalents, c'est-à-dire par la substitution d'un résidu par un autre possédant les mêmes caractéristiques. Ainsi, les substitutions classiques se font entre Ala, Val, Leu et De ; entre Ser et Thr ; entre les résidus acides Asp et Glu ; entre Asn et Gln ; et entre les résidus basiques Lys et Arg ; ou entre les résidus aromatiques Phe et Tyr.

Dans l'invention, le terme « acide aminé » se réfère ici à tout acide organique naturel ou non naturel ayant la formule :

-NHR-CR-C(O)-O-

où chaque -R est indépendamment sélectionné entre un hydrogène et un groupement alkyl ayant entre 1 et 12 atomes de carbone. Préférentiellement, au moins un groupement -R de chaque acide aminé est un hydrogène. Par le terme « alkyl », on entend ici une chaîne carbonée pouvant être linéaire ou ramifiée, substituée (mono- ou poly-) ou non-substituée ; saturée, mono-saturée (une double ou triple liaison dans la chaîne) ou poly-insaturée (deux ou plusieurs doubles liaisons, deux ou plusieurs triples liaisons, une ou plusieurs doubles liaisons et une ou plusieurs triples liaisons dans la chaîne).

Le terme « peptide » désigne un enchaînement de deux ou plusieurs acides aminés liés entre eux par des liaisons peptidiques ou par des liaisons peptidiques modifiées.

Par « peptide », il faut entendre le peptide naturel ou synthétique de l'invention tel que décrit ci-dessus, ou au moins l'un de ses fragments, qu'il soit obtenu par protéolyse ou de manière synthétique, ou encore tout peptide naturel ou synthétique dont la séquence est totalement ou partiellement constituée par la séquence du peptide précédemment décrit.

De façon à améliorer la résistance à la dégradation, il peut être nécessaire d'utiliser une forme protégée du peptide selon l'invention. La forme de protection doit évidemment être une forme biologiquement compatible et doit être compatible avec une utilisation dans le domaine des cosmétiques ou de la pharmacie.

De nombreuses formes de protection biologiquement compatibles peuvent être envisagées. Elles sont bien connues de l'homme du métier comme, par exemple, l'acylation ou l'acétylation de l'extrémité amino-terminale, ou l'amidation ou l'estérification de l'extrémité carboxy-terminale. Ainsi, l'invention concerne une composition telle que définie précédemment, caractérisée par le fait que le peptide de séquence SEQ ID n°1 à SEQ ID n°10 est sous forme protégée ou non. On peut utiliser une protection basée sur une substitution sur l'extrémité amino-terminale par un groupement acétyle, un groupement benzoyle, un groupement tosyle ou un groupement benzyloxycarbonyle. De préférence, on utilise une protection basée sur l'amidation de la fonction hydroxyle de l'extrémité carboxy-terminale par un groupement NYY avec Y représentant une chaîne alkyle de C₁ à C₄, ou l'estérification par un groupement alkyle. Il est également possible de protéger les deux extrémités du peptide.

Les dérivés de peptides concernent aussi les acides aminés et les peptides reliés entre eux par une liaison pseudo-peptidique. On entend par « liaison pseudo-peptidique », tous les types de liaisons susceptibles de remplacer les liaisons peptidiques « classiques ».

Dans le domaine des acides aminés, les molécules ont une géométrie telle, qu'elles peuvent théoriquement se présenter sous la forme d'isomères optiques différents. Il existe ainsi une conformation moléculaire de l'acide aminé (AA) qui dévie à droite le plan de polarisation de la lumière (conformation dextrogyre ou D-aa), et une conformation moléculaire de l'acide aminé (aa) qui dévie à gauche le plan de polarisation de la lumière (conformation lévogyre ou L-aa). Les acides aminés naturels sont toujours de conformation lévogyre, en conséquence, un peptide d'origine naturelle ne sera constitué que d'acides aminés de type L-aa. Cependant, la synthèse chimique en laboratoire permet de préparer des acides aminés ayant les deux conformations possibles. A partir de ce matériel de base, il est possible d'incorporer lors de la synthèse de peptide aussi bien des acides aminés sous forme d'isomères optiques dextrogyres que lévogyres. Ainsi, les acides aminés constituant le peptide selon l'invention peuvent être sous configuration L- et D- ; de manière préférentielle, les acides aminés sont sous forme L-. Le peptide selon l'invention peut donc être sous forme L-, D- ou DL-.

Le peptide de formule générale (I) selon l'invention peut être obtenu soit par synthèse chimique classique (en phase solide ou en phase homogène liquide), soit par synthèse enzymatique (Kullman et al., J. Biol. Chem. 1980, 225, 8234), à partir d'acides aminés constitutifs ou de leurs dérivés.

Le peptide selon l'invention peut également être obtenu par fermentation d'une souche de bactéries modifiées ou non, par génie génétique, ou encore par extraction de protéines d'origine animale ou végétale, préférentiellement d'origine végétale, suivie d'une hydrolyse contrôlée qui libère des fragments peptidiques correspondant totalement ou partiellement aux peptides de formule générale (I).

De très nombreuses protéines trouvées dans les plantes sont susceptibles de contenir ces séquences au sein de leur structure. L'hydrolyse ménagée permet de dégager ces fragments peptidiques. Il est possible, mais non nécessaire pour réaliser l'invention, d'extraire soit les protéines concernées d'abord et de les hydrolyser ensuite, soit d'effectuer l'hydrolyse d'abord sur un extrait brut et de purifier les fragments peptidiques ensuite. Il est également possible d'utiliser certains extraits hydrolysés sans en purifier les fragments peptidiques correspondant aux peptides de formule générale I selon l'invention, mais en s'assurant toutefois de la présence desdits fragments par des moyens analytiques appropriés.

D'autres procédés plus simples ou plus complexes peuvent être envisagés par l'homme du métier connaissant le métier de synthèse, d'extraction et de purification des protéines et des peptides. Ainsi, le peptide selon l'invention peut être d'origine naturelle ou synthétique. Préférentiellement selon l'invention, le peptide est obtenu par synthèse chimique.

Selon l'invention, l'agent actif peut être un peptide unique, un mélange de peptides ou de dérivés peptidiques et/ou constitués de dérivés d'acides aminés.

Selon un mode de réalisation avantageux de l'invention, l'agent actif selon l'invention est préalablement solubilisé dans un ou plusieurs solvants cosmétiquement ou pharmaceutiquement acceptables, classiquement utilisés par l'homme du métier, comme l'eau, le glycérol, l'éthanol, le propylène glycol, le butylène glycol, le dipropylène glycol, les diglycols éthoxylés ou propoxylés, les polyols cycliques, la vaseline, une huile végétale ou tout mélange de ces solvants.

Selon encore un autre mode de réalisation avantageux de l'invention, l'agent actif selon l'invention est préalablement solubilisé dans un vecteur cosmétique ou pharmaceutique, comme les liposomes, ou adsorbé sur des polymères organiques poudreux, des supports minéraux comme les talcs et bentonites, et plus généralement solubilisé dans, ou fixé sur, tout vecteur cosmétiquement ou pharmaceutiquement acceptable.

L'invention a pour second objet une composition cosmétique, nutraceutique ou pharmaceutique, et notamment dermatologique, comprenant, dans un milieu physiologiquement adapté, une quantité efficace d'un peptide de formule générale (I), en tant qu'agent actif, seul ou en association avec au moins un autre agent actif.

Il est bien évident que l'invention s'adresse aux mammifères en général, et plus particulièrement aux êtres humains.

La quantité efficace d'agent actif correspond à la quantité nécessaire pour obtenir le résultat recherché, à savoir, stimuler la régénération cutanée, améliorer la restructuration du derme et/ou prévenir ou lutter contre les manifestations du vieillissement cutané.

Selon un mode de réalisation avantageux de l'invention, l'agent actif selon l'invention est présent dans les compositions de l'invention à une concentration comprise entre 0,0005 et 500 ppm (parties par million, poids/poids) environ, et préférentiellement à une concentration comprise entre 0,01 et 5 ppm environ par rapport au poids total de la composition finale.

Il est bien entendu que l'agent actif selon l'invention peut être utilisé seul ou bien en association avec au moins un autre agent actif, dans une composition cosmétique ou nutraceutique, ou pour la préparation d'une composition pharmaceutique et/ou dermatologique.

La composition utilisable selon l'invention peut en particulier consister en une composition pour soins capillaires, et notamment un shampooing, un après-shampooing, une lotion traitante, une crème ou un gel coiffant, une lotion restructurante pour les cheveux, un masque, etc. La composition cosmétique selon l'invention peut être utilisée notamment dans les traitements mettant en oeuvre une application qui est suivie ou non d'un rinçage, ou encore sous forme de shampooing. Ainsi, l'agent actif selon l'invention pourra avantageusement être utilisé dans les soins antipelliculaires du cuir chevelu.

Elle peut également se présenter sous forme de teinture ou de mascara à appliquer au pinceau ou au peigne, en particulier sur les cils, les sourcils ou les cheveux.

Avantageusement, les compositions utilisables selon l'invention contiennent en outre divers agents actifs destinés à la prévention et/ou au traitement des désordres liés au vieillissement, tels que des actifs hydratants, éclaircissants, lissants ou antirides.

Les compositions selon l'invention pourront être appliquées par toute voie appropriée, notamment orale, parentérale ou topique externe, et leur formulation sera adaptée par l'homme du métier, en particulier pour des compositions cosmétiques ou dermatologiques. Avantageusement, les compositions selon l'invention sont destinées à une administration par voie topique cutanée. Ces compositions doivent donc contenir un milieu cosmétiquement et/ou dermatologiquement acceptable, c'est-à-dire compatible avec la peau et les phanères, et couvrent toutes les formes cosmétiques ou dermatologiques. Ces compositions pourront notamment être sous forme de crèmes, émulsions huile-dans-eau, ou eau-dans-huile ou émulsions multiples, solutions, suspensions, gels, laits, lotions, sticks ou encore de poudres, et adaptées à une application sur la peau, les lèvres et/ou les phanères. Ces compositions comprennent les excipients nécessaires à leur formulation, tels que solvants, épaississants, diluants, tensioactifs, anti-oxydants, colorants, conservateurs, parfums.

Selon une autre forme de l'invention, les compositions seront appropriées à une application topique à usage buccal. Ainsi, les compositions pourront notamment se présenter sous la forme d'une émulsion, d'un gel ou d'un spray, ou d'une gomme à mâcher, d'un bain de bouche, d'une pâte dentifrice, d'une colle pour denture ou d'un pansement buccal.

Selon une autre forme de l'invention, les compositions seront appropriées à une administration orale pour un usage pharmaceutique ou nutraceutique. Ainsi, les compositions pourront notamment se présenter sous forme de comprimés, capsules, gélules, pâtes à mâcher, poudres à consommer telles quelles ou à mélanger extemporanément avec un liquide, sirops, gels, et toute autre forme connue de l'homme du métier. Elles contiendront des excipients de formulation appropriés, tels que colorants, édulcorants, aromatisants, agents de charge, liants, conservateurs.

Ces compositions pourront notamment se présenter sous forme d'une solution aqueuse, hydroalcoolique ou huileuse ; d'une émulsion huile-dans-eau, eau-dans-huile ou émulsions multiples ; elles peuvent aussi se présenter sous forme de crèmes, de suspensions, ou encore de poudres, adaptées à une application sur la peau, les muqueuses, les lèvres et/ou les phanères. Ces compositions peuvent être plus ou moins fluides et avoir l'aspect d'une crème, d'une lotion, d'un lait, d'un sérum, d'une pommade, d'un gel, d'une pâte ou d'une mousse. Elles peuvent aussi se présenter sous forme solide, comme un stick, ou être appliquées sur la peau sous forme d'aérosol. Elles peuvent être utilisées comme produit de soin et/ou comme produit de maquillage de la peau.

Ces compositions comprennent, en outre, tout additif communément utilisé dans le domaine d'application envisagé ainsi que les adjuvants nécessaires à leur formulation, tels que des solvants, des épaississants, des diluants, des anti-oxydants, des colorants, des filtres solaires, des agents auto-bronzants, des pigments, des charges, des conservateurs, des parfums, des absorbeurs d'odeur, des actifs cosmétiques ou pharmaceutiques, des huiles essentielles, des vitamines, des acides gras essentiels, des tensioactifs, des polymères filmogènes, etc.

Dans tous les cas, l'homme de métier veillera à ce que ces adjuvants ainsi que leurs proportions soient choisis de telle manière à ne pas nuire aux propriétés avantageuses recherchées de la composition selon l'invention. Ces adjuvants peuvent, par exemple, correspondre à 0,01 à 20 % du poids total de la composition. Lorsque la composition de l'invention est une émulsion, la phase grasse peut représenter de 5 à 80 % en poids et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés en une proportion allant de 0,3 à 30 % en poids, par rapport au poids total de la composition.

L'invention a pour troisième objet l'utilisation de l'agent actif dans des compositions cosmétiques ou nutraceutiques, ou pour la préparation de compositions pharmaceutiques.

Ainsi, l'agent actif, grâce à ses propriétés particulières, pourra être utilisé dans une composition cosmétique ou nutraceutique destinée à prévenir et/ou lutter contre les manifestations du vieillissement cutané, à stimuler la régénération cutanée et à restructurer le derme.

D'autre part, l'agent actif selon l'invention pourra être utilisé avantageusement dans une composition cosmétique ou nutraceutique destinée à lutter de manière préventive et/ou curative contre les manifestations du vieillissement cutané, et plus spécifiquement, afin de lutter contre et/ou de prévenir le vieillissement photo-induit (photo-vieillissement). Ainsi, l'agent actif selon l'invention, ou la composition le contenant, permettra de stimuler la régénération du derme et de l'épiderme.

Selon un autre aspect de l'invention, l'agent actif selon l'invention pourra être utilisé avantageusement dans une composition cosmétique ou nutraceutique destinée à protéger la peau et les muqueuses contre tous types d'agressions extérieures.

On entend par l'expression « agression extérieure », les agressions que peut produire l'environnement. A titre d'exemple, on peut citer des agressions telles que la pollution, les UV, ou encore les produits à caractère irritant tels que les tensioactifs, les conservateurs ou les parfums. Par pollution, on entend aussi bien la pollution « extérieure » due par exemple aux particules de diesel, à l'ozone ou aux métaux lourds, que la pollution « intérieure » qui peut être due notamment aux émissions de solvants de peintures, de colles, ou de papier-peints (tels que toluène, styrène, xylène ou benzaldehyde), ou bien encore la fumée de cigarette. La présence d'une flore bactérienne active et les métabolites qu'elle peut produire sont également une source d'agression possible pour les muqueuses.

La muqueuse buccale, et particulièrement la gencive, est soumise a d'importantes agressions bactériennes ainsi qu'à un stress mécanique lié à la mastication. En conséquence, ces tissus sont le siège d'un renouvellement constant visant à maintenir une étroite cohésion entre le tissu minéralisé dentaire et le tissu conjonctif gingival.

Selon encore un autre aspect de l'invention, l'agent actif pourra être utilisé avantageusement dans une composition cosmétique ou nutraceutique, ou pour préparer une composition pharmaceutique destinée à stimuler la régénération de la muqueuse buccale et de la gencive.

L'invention a également pour objet l'utilisation dans une composition cosmétique, ou pour la préparation d'une composition pharmaceutique, d'une quantité efficace d'agent actif tel que décrit précédemment, l'agent actif, ou la composition le contenant, étant destiné à prévenir les dommages causés à la peau par une exposition au soleil ou à un environnement agressif.

L'invention consiste encore en l'utilisation de l'agent actif pour la préparation d'une composition pharmaceutique destinée à accélérer la cicatrisation et à prévenir ou traiter les pathologies liées à un défaut de cicatrisation de la peau et des muqueuses, telles que les ulcères de jambe, les récessions gingivales, les gingivites ou les ulcères cornéens.

Des modes de réalisation particuliers de ce procédé de traitement cosmétique résultent également de la description précédente. D'autres avantages et caractéristiques de l'invention apparaîtront mieux à la lecture des exemples donnés à titre illustratif et non limitatif.

### Exemple 1 : Mise en évidence de l'effet activateur du peptide SEQ ID n° 4 sur l'expression du collagène de type I

Le but de cette étude est de déterminer l'effet activateur du peptide SEQ ID n° 4 sur l'expression du collagène de type I dans des fibroblastes humains normaux. La quantité de collagène I exprimée est évaluée par immunofluorescence sur cellules cultivées *in vitro* et sur culture *ex vivo* de peaux.

### Protocole:

### - Culture in vitro :

Des fibroblastes humains normaux cultivés dans des chambres de culture multi-puits Lab-Tek™ sont mis en présence du peptide SEQ ID n° 4 à 1 % à partir d'une solution mère à 100 ppm, pendant 48 heures, à raison de 2 applications chaque 24 heures. Des contrôles non traités sont réalisés.

Les cultures cellulaires sont ensuite fixées en vue d'un immunomarquage.

### - Culture ex vivo :

Des échantillons de peau humaine sont mis en culture à l'interface air/liquide. Une solution à 1 % d'une solution mère à 100 ppm de peptide SEQ ID n° 4 est appliquée topiquement, puis les échantillons sont incubés durant 48 et 72 heures. Des contrôles non traités sont réalisés. Ces échantillons de peau sont ensuite fixés avec du formaldéhyde puis inclus dans la paraffine. Des coupes de 2 à 3 µm sont alors réalisées. L'immunomarquage est effectué après différentes étapes de lavage et d'incubation de ces coupes.

### - Immunomarquage :

L'immunomarquage du collagène **I** est réalisé à l'aide d'un anticorps polyclonal spécifique obtenu chez le lapin (TEBU). La réaction est révélée par un anticorps secondaire couplé à un marqueur fluorescent (ALEXA Fluor 488). Les cellules et les coupes de peau sont alors examinées au microscope à Epi-fluorescence (Nikon Eclipse E600 microscope).

Résultats : Les observations microscopiques montrent une fluorescence plus forte dans les cellules traitées par le peptide SEQ ID n° 4 à 1%, ainsi que dans le derme des peaux traitées.

Conclusion : Le peptide SEQ ID n° 4 stimule fortement l'expression du collagène de type I dans les fibroblastes humains cultivés en condition *in vitro* et *ex vivo.*

### Exemple 2 : Mise en évidence de l'effet activateur du peptide SEQ ID n° 5 sur l'expression du collagène de type III

Le but de cette étude est de déterminer l'effet activateur du peptide SEQ ID n° 5 sur l'expression du collagène de type III dans des fibroblastes humains normaux. La quantité de collagène III exprimée est évaluée par immunofluorescence sur cellules cultivées *in vitro* et sur culture *ex vivo* de peaux.

### Protocole :

### - Culture in vitro :

Des fibroblastes humains normaux cultivés dans des chambres de culture multi-puits Lab-Tek™ sont mis en présence du peptide SEQ ID n° 5 à 1 % à partir d'une solution mère à 100 ppm, pendant 48 heures, à raison de 2 applications chaque 24 heures. Des contrôles non traités sont réalisés.

Les cultures cellulaires sont ensuite fixées en vue d'un immunomarquage.

### - Culture ex vivo :

Des échantillons de peau humaine sont mis en culture à l'interface air/liquide. Une solution à 1 % d'une solution mère à 100 ppm de peptide SEQ ID n° 5 est appliquée topiquement, puis les échantillons sont incubés durant 48 et 72 heures. Des contrôles non traités sont réalisés. Ces échantillons de peau sont ensuite fixés avec du formaldéhyde puis inclus dans la paraffine. Des coupes de 2 à 3 µm sont alors réalisées.
L'immunomarquage est effectué après différentes étapes de lavage et d'incubation de ces coupes.

### - Immunomarquage :

L'immunomarquage du collagène III est réalisé à l'aide d'anticorps polyclonaux spécifiques obtenu chez le lapin (TEBU). La réaction est révélée par un anticorps secondaire couplé à un marqueur fluorescent (ALEXA Fluor 488). Les cellules et les coupes de peau sont alors examinées au microscope à Epi-fluorescence (Nikon Eclipse E600 microscope).

Résultats : Les observations microscopiques montrent une fluorescence plus forte dans les cellules traitées par le peptide SEQ ID n° 5 à 1 %, ainsi que dans le derme des peaux traitées.

Conclusion : Le peptide SEQ ID n° 5 stimule fortement l'expression du collagène de type III dans les fibroblastes humains cultivés en condition *in vitro* et *ex vivo.*

### Exemple 3: Mise en évidence de l'effet activateur du peptide SEQ ID n° 5 sur l'expression de la fibronectine

Le but de cette étude est de déterminer l'effet activateur du peptide SEQ ID n° 5 sur l'expression de la fibronectine dans les fibroblastes humains. L'évaluation de la quantité de fibronectine est réalisée par immunofluorescence.

### Protocole:

Des fibroblastes humains cultivés dans des chambres de culture multi-puits Lab-Tek™ sont mis en présence du peptide SEQ ID n° 5 à 1 % à partir d'une solution mère à 100 ppm, pendant 48 heures, à raison de 2 applications chaque 24 heures. Des contrôles non traités sont réalisés.

Les cultures cellulaires sont ensuite fixées en vue d'un immunomarquage.

L'immunomarquage de la fibronectine est réalisé à l'aide d'anticorps polyclonaux spécifiques obtenu chez le lapin (TEBU). La réaction est révélée par un anticorps secondaire couplé à un marqueur fluorescent (ALEXA Fluor 488).

Les cellules sont examinées au microscope à Epi-fluorescence (Nikon Eclipse E600 microscope).

Résultats : Les observations microscopiques montrent une fluorescence plus forte dans les kératinocytes traités par le peptide SEQ ID n° 5 à 1 %.

Conclusion : Le peptide SEQ ID n° 5 stimule fortement l'expression de la fibronectine dans les fibroblastes humains normaux.

### Exemple 4: Mise en évidence de l'effet activateur du peptide SEQ ID n° 5 sur l'expression des kératines

Le but de cette étude est de déterminer l'influence du peptide SEQ ID n° 5 sur l'expression des kératines. La quantité de protéine a été évaluée par immunofluorescence sur des coupes de peau humaine.

Protocole : Des échantillons de peau humaine sont mis en culture à l'interface air/liquide. Une solution à 1 % d'une solution mère à 100 ppm de peptide SEQ ID n° 5 est appliquée topiquement, puis les échantillons sont incubés durant 48 heures.

Ces échantillons de peau sont ensuite fixés avec du formaldéhyde puis inclus dans la paraffine. Des coupes de 2 à 3 µm sont alors réalisées. L'immunomarquage est effectué après différentes étapes de lavage et d'incubation de ces coupes.

L'immunomarquage des kératines est réalisé à l'aide d'un anticorps monoclonal spécifique (NOVOCASTRA), puis d'un anticorps secondaire couplé à un marqueur fluorescent (ALEXA Fluor 488 donkey anti-mouse IgG, A21202, Molecular Probes, 1/1000^{ème}).

Les coupes de peau sont alors examinées au microscope à Epi-fluorescence (Nikon Eclipse E600 microscope).

Résultats : Les observations microscopiques montrent une fluorescence plus forte dans les peaux traitées par le peptide SEQ ID n° 5 à 1 %, en particulier les couches supérieures de l'épiderme.

Conclusions : Le peptide SEQ ID n° 5, à la concentration de 0,05 ppm, stimule fortement l'expression des kératines.

### Exemple 5 : Mise en évidence de l'effet activateur du peptide SEQ ID n° 5 sur l'expression de l'intégrine bêta 1

Le but de cette étude est de déterminer l'effet activateur du peptide SEQ ID n° 5 sur l'expression de l'intégrine bêta 1 dans des kératinocytes humains HaCaT. La quantité d'intégrine bêta 1 exprimée est évaluée par immunofluorescence sur cellules cultivées *in vitro* et sur culture *ex vivo* de peaux.

### Protocole:

### - Culture in vitro :

Des kératinocytes humains HaCaT cultivés dans des chambres de culture multi-puits Lab-Tek™ sont mis en présence du peptide SEQ ID n° 5 à 1 % à partir d'une solution mère à 100 ppm, pendant 48 heures, à raison de 2 applications chaque 24 heures. Des contrôles non traités sont réalisés.

Les cultures cellulaires sont ensuite fixées en vue d'un immunomarquage.

### - Culture ex vivo :

Des échantillons de peau humaine sont mis en culture à l'interface air/liquide. Une solution à 1 % d'une solution mère à 100 ppm de peptide SEQ ID n° 5 est appliquée topiquement, puis les échantillons sont incubés durant 48 et 72 heures. Des contrôles non traités sont réalisés. Ces échantillons de peau sont ensuite fixés avec du formaldéhyde puis inclus dans la paraffine. Des coupes de 2 à 3 µm sont alors réalisées. L'immunomarquage est effectué après différentes étapes de lavage et d'incubation de ces coupes.

### - Immunomarquage :

L'immunomarquage de l'intégrine bêta 1 est réalisé à l'aide d'un anticorps monoclonal spécifique (TEBU). La réaction est révélée par un anticorps secondaire couplé à un marqueur fluorescent (ALEXA Fluor 488 donkey anti-mouse IgG, A21202, Molecular Probes, 1/1000^{ème}). Les cellules et les coupes de peau sont alors examinées au microscope à Epi-fluorescence (Nikon Eclipse E600 microscope).

Résultats : Les observations microscopiques montrent une fluorescence plus forte dans les cellules traitées par le peptide SEQ ID n° 5 à 1 %, ainsi que dans l'épiderme des peaux traitées.

Conclusions : Le peptide SEQ ID n° 5 stimule fortement l'expression de l'intégrine bêta 1 dans les kératinocytes humains HaCaT humains cultivés en condition *in vitro,* ainsi que dans l'épiderme cultivé en condition *ex vivo.*

### Exemple 6 : Préparation de compositions

### 1 - Crème de jour

| ***Noms commerciaux*** | ***Noms INCI*** | ***% massique*** |
|---|---|---|
| ***PHASE A*** | | |
| MONTANOV 68 | Cetearyl Alcohol (and) Cetearyl Glucoside | 5,00 |
| HUILE DE JOJOBA | Simmondsia Chinensis (Jojoba) Seed Oil | 3,00 |
| HUILE DE VASELINE | Paraffinum Liquidum (Mineral Oil) | 2,00 |
| SQUALANE | Squalane | 3,00 |
| CERAPHYL 368 | Ethylhexyl palmitate | 4,00 |
| CERAPHYL41 | C12-C15 Alkyl Lactate | 3,00 |
| RAPITHIX A-60 | Sodium polyacrylate (and) Hydrogenated Polydecene (and) Trideceth -6 | 0,30 |

| ***PHASE B*** | | |
|---|---|---|
| GLYCERINE | Glycerin | 5,00 |
| ALLANTOÏNE | Allantoin | 0,10 |
| EAU DEMINERALISEE | Aqua (Water) | qsp 100 |

| ***PHASE* C** | | |
|---|---|---|
| ROKONSAL MEP | Phenoxyethanol (and) Methylparaben (and) Ethylparaben (and) Propylparaben | 0,50 |

| ***PHASE* D** | | |
|---|---|---|
| PEPTIDE SEQ ID n° 5 | | 0,1 ppm |

| ***PHASE E*** | | |
|---|---|---|
| PARFUM | Parfum (Fragrance) | qsp |

### Mode Opératoire :

Peser les ingrédients de la phase grasse et chauffer à 70°C sous agitation. Préparer la phase B et la chauffer à 70°C. Emulsionner la phase A dans la phase B. Ajouter la phase D vers 50°C sous agitation. En dessous de 40°C, additionner l'agent actif (Phase D). Parfumer et refroidir jusqu'à température ambiante.

### 2 -Crème anti-âge :

| ***Noms commerciaux*** | ***Noms INCI*** | ***% massique*** |
|---|---|---|
| ***Phase A*** | | |
| Montanov 68 | Cetearyl Alcohol (and) Cetearyl Glucoside | 6,00 |
| Squalane | Squalane | 3,00 |
| Cetiol SB 45 | Butyrospermum Parkii (Shea Butter) | 2,00 |
| Waglinol 250 | Cetearyl Ethylhexanoate | 3,00 |
| Amerchol L- 101 | Mineral Oil (and) Lanolin Alcohol | 2,00 |
| Abil 350 | Dimethicone | 1,50 |
| BHT | BHT | 0,01 |
| Coenzyme Q10 | Ubiquinone | 0,10 |

| ***Phase B*** | | |
|---|---|---|
| Huile d'Avocat | Persea Gratissima (Avocado) Oil | 1,25 |
| Phenonip | Phenoxyethanol (and) Methylparaben (and) Ethylparaben (and) Butylparaben (and) Propylparaben (and) Isobutylparaben | 0,75 |

| ***Phase C*** | | |
|---|---|---|
| Eau déminéralisée | Aqua (Water) | qsp |
| Butylene Glycol | Butylene Glycol | 2,00 |
| Glucam E10 | Methyl Gluceth-10 | 1,00 |
| Allantoin | Allantoin | 0,15 |
| Carbopol Ultrez 10 | Carbomer | 0,20 |

| ***Phase D*** | | |
|---|---|---|
| TEA | Triethanolamine | 0,18 |

| ***Phase E*** | | |
|---|---|---|
| Peptide SEQ ID n° 5 | | 1 ppm |
| GP4G | Water (and) Artemia Extract | 1,50 |
| Collaxyl | Water (and) Butylene Glycol (and) Hexapeptide-9 | 3,00 |

| ***Phase F*** | | |
|---|---|---|
| Parfum | Parfum (Fragrance) | qsp |
| Colorant | | qsp |

### Mode Opératoire :

Préparer et fondre la phase A à 65-70°C. Chauffer la phase C à 65-70°C. La phase B est ajoutée à la phase A juste avant d'émulsionner A dans B. A environ 45°C, le carbomer est neutralisé par addition de la phase D. La phase E est ensuite additionnée sous légère agitation et le refroidissement est poursuivi jusqu'à 25°C. La phase F est alors additionnée si souhaité.

### 3 -Crème protectrice de jour :

| ***Noms commerciaux*** | ***Noms INCI*** | ***% massique*** |
|---|---|---|
| ***Phase A*** | | |
| Emulium Delta | Cetyl alcohol (and) Glyceryl Stearate (and) PEG-75 Stearate (and) Ceteth-20 (and) Steareth-20 | 4,00 |
| Lanette O | Cetearyl Alcohol | 1,50 |
| D C 200 Fluid/100cs | Dimethicone | 1,00 |
| DUB 810C | Coco Caprylate/Caprate | 1,00 |
| DPPG | Propylene Glycol Dipelargonate | 3,00 |
| DUB DPHCC | Dipentaerythrityl Hexacaprylate/Hexacaprate | 1.50 |
| Cegesoft PS6 | Vegetable Oil | 1,00 |
| Vitamine E | Tocopherol | 0,30 |
| Phenonip | Phenoxyethanol (and) Methylparaben (and) Ethylparaben (and) Butylparaben (and) Propylparaben (and) Isobutylparaben | 0,70 |

| ***Phase B*** | | |
|---|---|---|
| Eau déminéralisée | Aqua | qsp 100 |
| Glycerine | Glycerin | 2,00 |
| Carbopol EDT 2020 | Acrylates/C10-30Alkyl Acrylate Crosspolymer | 0,15 |
| Keltrol BT | Xanthan Gum | 0,30 |

| ***Phase C*** | | |
|---|---|---|
| Sodium Hydroxide (sol.à 10%) | Sodium Hydroxide | 0,30 |

| ***Phase D*** | | |
|---|---|---|
| Eau déminéralisée | Aqua | 5,00 |
| Stay-C 50 | Sodium Ascorbyl Phosphate | 0,50 |

| ***Phase E*** | | |
|---|---|---|
| Butylene Glycol | Butylene Glycol | 2,00 |
| Dekaben CP | Chlorphenesin | 0,20 |

| ***Phase F*** | | |
|---|---|---|
| GP4G | Water (and) Artemia Extract | 1,00 |
| Peptide SEQ ID n° 5 | | 5 ppm |

### Mode Opératoire :

Préparer la phase A et chauffer à 75°C sous agitation. Préparer la phase B en dispersant le carbopol, puis la gomme xanthane sous agitation. Laisser reposer. Chauffer à 75°C.

A température, émulsionner A dans B sous agitation rotor-stator. Neutraliser avec la phase C sous agitation rapide. Après refroidissement à 40°C, additionner la phase D, puis la phase E. Le refroidissement est poursuivi sous agitation légère et la phase F rajoutée.

## Revendications

1. Peptide de formule générale (I)
R₁-(AA)ₙ-Ile-X₁-Leu-X₂-Phe-Leu-X₃.(AA)ₚ-R₂
dans laquelle,
X₁, X₂. X₃ est l'arginine, la lysine ou l'histidine,
AA représente un acide aminé quelconque, autre que l'asparagine ou la glutamine, ou un de ses dérivés, et n et p sont des nombres entiers compris entre 0 et 4,
R₁ représente la fonction amine primaire de l'acide aminé N-terminal, libre ou substituée par un groupement protecteur pouvant être choisi parmi un groupement acétyle, un groupement benzoyle, un groupement tosyle ou un groupement benzyloxycarbonyle,
R₂ représente le groupe hydroxyle de la fonction carboxyle de l'acide aminé C-terminal, libre ou substitué par un groupement protecteur pouvant être choisi parmi une chaîne alkyle de C₁ à C₂₀, ou un groupement NH2, NHY ou NYY avec Y représentant une chaîne alkyle de C₁ à C₄,
ladite séquence de formule générale (I) pouvant comprendre des substitutions des acides aminés X₁ à X₃ par d'autres acides aminés chimiquement équivalents.

2. Peptide selon les revendications 1 **caractérisé en ce qu'**il correspond à une des séquences suivantes :
(SEQ ID n°4) Ile-Arg-Leu-Arg-Phe-Leu-Arg
(SEQ ID n°5) Ile-Arg-Leu-Arg-Phe-Leu-Arg -NHY
(SEQ ID n°6) Ile-Lys-Leu-Arg-Phe-Leu-His
(SEQ ID n°7) Ile-Lys-Leu-Arg-Phe-Leu-His-NH₂
(SEQ ID n°8) Ile-His -Leu-Lys-Phe-Leu-Arg
(SEQ ID n°9) Ile-His-Leu-Lys-Phe-Leu-Arg-NH₂
(SEQ ID n°10) Ile-Lys-Leu-His-Phe-Leu-Lys

3. Peptide selon la revendication 2 **caractérisé en ce qu'**il correspond à la séquence SEQ ID n°4.

4. Peptide selon la revendication 2 **caractérisé en ce qu'**il correspond à la séquence SEQ ID n°5.

5. Peptide selon l'une quelconque des revendications précédentes, **caractérisé en ce qu'**il possède au moins un groupement fonctionnel protégé par un groupement protecteur, ce groupement protecteur étant soit une acylation ou une acétylation de l'extrémité amino-terminale, soit une amidation ou une estérification de l'extrémité carboxy-terminale, soit les deux.

6. Composition cosmétique ou pharmaceutique **caractérisée en ce qu'**elle contient dans un milieu physiologiquement acceptable, en tant qu'agent actif, une quantité efficace de peptide définit selon les revendications 1 à 5, préalablement solubilisé dans un ou plusieurs solvants cosmétiquement ou pharmaceutiquement acceptables, comme l'eau, le glycérol, l'éthanol, le propylène glycol, le butylène glycol, le dipropylène glycol, les diglycols éthoxylés ou propoxylés, les polyols cycliques, la vaseline, une huile végétale ou tout mélange de ces solvants.

7. Composition cosmétique ou pharmaceutique selon la revendication 6, **caractérisée en ce que** ledit peptide est présent à une concentration comprise entre 0,0005 et 500 ppm environ, et préférentiellement à une concentration comprise entre 0,01 et 5 ppm.

8. Composition selon l'une des revendications 6 ou 7, **caractérisée en ce qu'**elle contient en outre un agent actif hydratant, et/ou un agent actif antiride, et/ou un agent actif éclaircissant.

9. Utilisation d'une quantité efficace de peptide, tel que défini dans l'une quelconque des revendications 1 à 5, en tant qu'agent actif, seul ou en association avec au moins un autre agent actif, dans une composition cosmétique ou nutraceutique destinée à prévenir et/ou lutter contre les manifestations du vieillissement cutané et/ou du photo-vieillissement cutané.

10. Utilisation d'une quantité efficace de peptide, tel que défini dans l'une quelconque des revendications 1 à 5, en tant qu'agent actif, seul ou en association avec au moins un autre agent actif, dans une composition cosmétique ou nutraceutique, destinée à stimuler la régénération cutané.

11. Utilisation d'une quantité efficace de peptide, tel que défini dans l'une quelconque des revendications 1 à 5, en tant qu'agent actif, seul ou en association avec au moins un autre agent actif, dans une composition cosmétique ou nutraceutique destinée à prévenir les dommages causés à la peau par une exposition au soleil ou à un environnement agressif, et/ou à protéger la peau et les muqueuses des agressions extérieures.

12. Utilisation d'une quantité efficace de peptide, tel que défini dans l'une quelconque des revendications 1 à 5, en tant qu'agent actif, seul ou en association avec au moins un autre agent actif, dans une composition cosmétique ou nutraceutique destinée à restructurer le derme et la matrice extracellulaire.

13. Utilisation d'une quantité efficace de peptide, tel que défini dans l'une quelconque des revendications 1 à 5, en tant qu'agent actif, seul ou en association avec au moins un autre agent actif, pour préparer une composition pharmaceutique destinée à accélérer la cicatrisation ou à prévenir ou traiter les pathologies liées à un défaut de cicatrisation de la peau et des muqueuses, telles que les ulcères de jambe, les récessions gingivales, les gingivites et les ulcères cornéens.

## Claims

1. A peptide of the following general formula (I):
R₁- (AA)ₙ-Ile-X₁-Leu-X₂-Phe-Leu-X₃-(AA)ₚ-R₂
in which
X₁, X₂, X₃ is arginine, lysine or histidine,
AA represents any amino acid, other than asparagine or glutamine, or a derivative thereof, and n and p are integers between 0 and 4,
R₁ represents the primary amino function of the N-terminal amino acid, whether free or substituted with a protective group which may be selected from an acetyl group, a benzoyl group, a tosyl group or a benzyloxycarbonyl group,
R₂ represents the hydroxyl group of the C-terminal amino acid's carboxyl function, whether free or substituted with a protective group which may be selected from a C₁-C₂₀ alkyl chain or a NH2, NHY or NYY group, where Y represents a C₁-C₄ alkyl chain,
wherein said sequence of general formula (I) may comprise substitutions of the amino acids X₁ to X₃ with other chemically equivalent amino acids.

2. The peptide according to claim 1, **characterized in that** it corresponds to the following sequences:
(SEQ ID No 4) Ile-Arg-Leu-Arg-Phe-Leu-Arg
(SEQ ID No 5) Ile-Arg-Leu-Arg-Phe-Leu-Arg-NH₂
(SEQ ID No 6) Ile-Lys-Leu-Arg-Phe-Leu-His
(SEQ ID No 7) Ile-Lys-Leu-Arg-Phe-Leu-His-NH₂
(SEQ ID No 8) Ile-His-Leu-Lys-Phe-Leu-Arg
(SEQ ID No 9) Ile-His-Leu-Lys-Phe-Leu-Arg-NH₂
(SEQ ID No 10) Ile-Lys-Leu-His-Phe-Leu-Lys.

3. The peptide according to claim 2, **characterized in that** it corresponds to the sequence SEQ ID No 4.

4. The peptide according to claim 2, **characterized in that** it corresponds to the sequence SEQ ID No 5.

5. The peptide according to any one of the preceding claims, **characterized in that** it has at least one functional group protected by a protecting group, wherein this protecting group is either an acylation or acetylation of the amino terminus, or an amidation or esterification of the carboxy terminus, or both.

6. A cosmetic or pharmaceutical composition **characterized in that** it contains, in a physiologically acceptable medium, as an active ingredient, an effective amount of a peptide as defined according to claims 1 to 5, previously solubilized in one or more cosmetically or pharmaceutically acceptable solvents, such as water, glycerol, ethanol, propylene glycol, butylene glycol, dipropylene glycol, ethoxylated or propoxylated diglycols, cyclic polyols, petrolatum, a vegetable oil or any mixture of these solvents.

7. The cosmetic or pharmaceutical composition according to claim 6, **characterized in that** said peptide is present at a concentration of between 0.0005 and 500 ppm, preferably at a concentration of between 0.01 and 5 ppm.

8. The composition according to one of claims 6 or 7, **characterized in that** it further contains a hydrating active agent, and/or an anti-wrinkle active agent, and/or an active lightening agent.

9. Use of an effective amount of the peptide as defined in any one of claims 1 to 5, as an active agent, alone or in combination with at least one other active agent, in a cosmetic or nutraceutical composition intended to prevent and/or combat the signs of skin aging and/or skin photo-aging.

10. Use of an effective amount of the peptide as defined in any one of claims 1 to 5, as an active agent, alone or in combination with at least one other active agent, in a cosmetic or nutraceutical composition, intended to stimulate skin regeneration.

11. Use of an effective amount of the peptide as defined in any one of claims 1 to 5, as an active agent, alone or in combination with at least one other active agent, in a cosmetic or nutraceutical composition intended to prevent damage to the skin caused by exposure to sunlight or to aggressive environments, and/or to protect the skin and mucous membranes from external aggressions.

12. Use of an effective amount of the peptide as defined in any one of claims 1 to 5, as an active agent, alone or in combination with at least one other active agent, in a cosmetic or nutraceutical composition intended for restructuring the dermis and extracellular matrix.

13. Use of an effective amount of the peptide as defined in any one of claims 1 to 5, as an active agent, alone or in combination with at least one other active agent, to prepare a pharmaceutical composition intended to accelerate healing or to prevent or treat diseases related to inadequate healing of the skin and mucous membranes, such as leg ulcers, gingival recession, gingivitis and corneal ulcers.

## Patentansprüche

1. Peptid der folgenden allgemeinen Formel (I):
R₁-(AA)ₙ-Ile-X₁-Leu-X₂-Phe-Leu-X₃-(AA)ₚ-R₂
worin
X₁, X₂, X₃ Arginin, Lysin oder Histidin ist,
AA eine beliebige Aminosäure darstellt, ausgenommen Asparagin oder Glutamin, oder ein Derivat davon, und n und p ganze Zahlen zwischen 0 und 4 sind,
R₁ die primäre Aminofunktion des N-terminalen Aminosäure darstellt, entweder frei oder substituiert mit einer Schutzgruppe, die aus einer Acetylgruppe, einer Benzoylgruppe, eine Tosylgruppe oder eine Benzyloxycarbonyl-Gruppe ausgewählt sein kann,
R₂ die Hydroxylgruppe der Carboxylfunktion des C-terminalen Aminosäure darstellt, entweder frei oder substituiert mit einer Schutzgruppe, die aus einem C₁-C₂₀-Alkylkette oder eine NH2, NHY oder NYY Gruppe ausgewählt sein können, wobei Y ein C₁-C₄-Alkyl Kette darstellt,
wobei die Sequenz der allgemeinen Formel (I) kann Substitutionen der Aminosäuren X₁ bis X₃ mit anderen chemisch äquivalenten Aminosäuren umfassen.

2. Peptid nach Anspruch 1, **dadurch gekennzeichnet, dass** es den folgenden Sequenzen entspricht:
(SEQ ID Nr. 4) Ile-Arg-Leu-Arg-Phe-Leu-Arg
(SEQ ID Nr. 5) Ile-Arg-Leu-Arg-Phe-Leu-Arg-NH₂
(SEQ ID Nr. 6) Ile-Lys-Leu-Arg-Phe-Leu-His
(SEQ ID Nr. 7) Ile-Lys-Leu-Arg-Phe-Leu-His-NH₂
(SEQ ID Nr. 8) Ile-His-Leu-Lys-Phe-Leu-Arg
(SEQ ID Nr. 9) Ile-His-Leu-Lys-Phe-Leu-Arg-NH₂
(SEQ ID Nr. 10) Ile-Lys-Leu-His-Phe-Leu-Lys.

3. Peptid nach Anspruch 2, **dadurch gekennzeichnet, dass** es der Sequenz SEQ ID Nr. 4 entspricht.

4. Peptid nach Anspruch 2, **dadurch gekennzeichnet, dass** es der Sequenz SEQ ID Nr. 5 entspricht.

5. Peptid nach einem der vorhergehenden Ansprüche, **dadurch gekennzeichnet, dass** es mindestens eine funktionelle Gruppe aufweist, welche durch eine Schutzgruppe geschützt ist, wobei diese Schutzgruppe entweder eine Acylierung oder Acetylierung der Amino-Terminus, oder eine Amidierung oder Veresterung der Carboxy-terminus, oder die beide ist.

6. Kosmetische oder pharmazeutische Zusammensetzung, **dadurch gekennzeichnet, dass** es, in einem physiologisch akzeptablen Medium, als einen aktiven Bestandteil, eine wirksame Menge des Peptids enthält, wie in den Ansprüchen 1 bis 5 definiert, zuvor in einem oder mehreren kosmetisch oder pharmazeutisch akzeptablen Lösungsmitteln solubilisiert, wie Wasser, Glycerin, Ethanol, Propylenglykol, Butylenglykol, Dipropylenglykol, ethoxylierten oder propoxylierten Diglykolen, zyklische Polyolen, Vaseline, pflanzliche Öle oder eine beliebige Mischung dieser Lösungsmittel.

7. Kosmetische oder pharmazeutische Zusammensetzung nach Anspruch 6, **dadurch gekennzeichnet, dass** das Peptid in einer Konzentration von zwischen 0,0005 und 500 ppm, vorzugsweise in einer Konzentration von zwischen 0,01 und 5 ppm vorliegt.

8. Zusammensetzung nach einem der Ansprüche 6 oder 7, **dadurch gekennzeichnet, dass** sie ferner einen hydratisierenden Wirkstoff und/oder einen Anti-Falten-Wirkstoff, und/oder einen aufhellenden Wirkstoff enthält.

9. Verwendung einer wirksamen Menge des Peptids, wie in einem der Ansprüche 1 bis 5 definiert, als Wirkstoff, allein oder in Kombination mit mindestens einem weiteren Wirkstoff in einer kosmetischen oder nutrazeutischen Zusammensetzung, die zur Verhinderung und/oder zur Bekämpfung der Zeichen der Hautalterung und/oder Lichtalterung der Haut geeignet ist.

10. Verwendung einer wirksamen Menge des Peptids, wie in einem der Ansprüche 1 bis 5 definiert, als Wirkstoff, allein oder in Kombination mit mindestens einem weiteren Wirkstoff in einer kosmetischen oder nutrazeutischen Zusammensetzung, die dazu bestimmt um die Regeneration der Haut anzuregen.

11. Verwendung einer wirksamen Menge des Peptids, wie in einem der Ansprüche 1 bis 5 definiert, als Wirkstoff, allein oder in Kombination mit mindestens einem weiteren Wirkstoff in einer kosmetischen oder nutrazeutischen Zusammensetzung, die zur Verhinderung der durch Bestrahlung mit Sonnenlicht oder aggressiven Umgebungen hervorgerufen Schädigung der Haut geeignet ist, und/oder um die Haut und die Schleimhäute vor äußeren Einflüssen zu schützen.

12. Verwendung einer wirksamen Menge des Peptids, wie in einem der Ansprüche 1 bis 5 definiert, als Wirkstoff, allein oder in Kombination mit mindestens einem weiteren Wirkstoff in einer kosmetischen oder nutrazeutischen Zusammensetzung für die Restrukturierung der Lederhaut und extrazelluläre Matrix.

13. Verwendung einer wirksamen Menge des Peptids, wie in einem der Ansprüche 1 bis 5 definiert, als Wirkstoff, alleine oder in Kombination mit mindestens einem anderen Wirkstoff, zur Herstellung einer pharmazeutischen Zusammensetzung zur Beschleunigung der Heilung oder zur Verhütung oder Behandlung von Krankheiten im Zusammenhang mit schlechter Heilung der Haut und Schleimhäute, wie Ulcus cruris, Zahnfleischschwund, Gingivitis und Hornhautulzera.
